# EUROPÄISCHE PATENTSCHRIFT

(11) **EP 0 946 432 B1**
(45) Veröffentlichungstag und Bekanntmachung des Hinweises auf die Patenterteilung: **10.01.2001**
(21) Anmeldenummer: 97951082.3
(22) Anmeldetag: 14.11.1997
(51) Int. Cl.: C02F 3/28, C02F 3/32

(54) **VERFAHREN ZUR REINIGUNG VON ABWASSER**
WASTE WATER PURIFICATION PROCESS
PROCEDE D'EPURATION D'EAUX USEES

(30) Priorität: 26.11.1996 DE 19648860
(43) Veröffentlichungstag der Anmeldung: 06.10.1999
(73) Patentinhaber: Dobelmann, Jan, Kai, 76131 Karlsruhe (DE)
(72) Erfinder: Dobelmann, Jan, Kai, 76131 Karlsruhe (DE)
(74) Vertreter: Bröseke, Eribert, Dipl.-Ing.
(86) Internationale Anmeldenummer: DE9702716
(87) Internationale Veröffentlichungsnummer: WO9823541

(56) Entgegenhaltungen:
- WO-A-93/06050
- US-A- 4 415 450
- US-A- 4 439 315
- US-A- 4 692 249
- US-A- 5 137 625

## Beschreibung

Die Erfindung betrifft ein Verfahren zur Behandlung von Abwasser in einem zweistufigen Verfahren bei gleichzeitiger Gewinnung von Energie in Form von Biogas.

Für die anaerobe Abwasserreinigung sind verschiedene Verfahren und Reaktoren diverser Bauarten bekannt, wie UASB, Festbettreaktoren, anaerobe Belebungsbecken u. a.

Diese Reaktoren werden bevorzugt bei organisch hochbelasteten Industrieabwässern eingesetzt.

Der Erfindung liegt die Aufgabe zugrunde, ein Verfahren zu entwickeln, das es erlaubt, organisch gering belastetes Abwasser (CSB < 500mg/l) einer energieerzeugenden Behandlungsmethode durch anaerobe Gärung zugängig zu machen.

Erfindungsgemäß wird die Aufgabe durch die kennzeichnenden Merkmale des Anspruches 1 gelöst. Bei dem erfindungsgemäßen Verfahren, das mit einer zweistufigen Verfahrensführung arbeitet und aus einer anaeroben Behandlung mit der Produktion von methanhaltigem Biogas als erster Reinigungsstufe und einer Pflanzenkläranlage als zweiter Reinigungsstufe besteht, wird die in der Pflanzenkläranlage geerntete Biomasse vor der ersten Reinigungsstufe in das zu behandelnde Abwasser eingeführt. Neben der in der zweiten Reinigungsstufe geernteten Biomasse können noch zusätzlich andere organische oder anorganische Stoffe und Abfälle in das Abwasser vor der ersten Reinigungsstufe eingebracht werden.

Durch das erfindungsgemäße Verfahren wird es möglich, daß organisch gering belastete Abwässer der anaeroben Methangärung zugänglich gemacht werden.

Bevor die in der zweiten Stufe anfallende Biomasse oder die zusätzlichen anderen organischen oder anorganischen Stoffe und Abfälle vor der ersten Stufe in das Abwasser eingeführt werden, werden sie durch Zerkleinern und Zerfasern in eine für die Vergärung notwendige Form aufbereitet. Für die Zerfaserung und Zerkleinerung der Biomasse und der anderen Abfälle werden an sich bekannte Systeme, wie System FIMA System, ULTRA TURAX u. ä., eingesetzt. Durch die gewollte Zuführung von in der zweiten Stufe anfallender Biomasse in das Abwasser vor der ersten Stufe wird die Belastung des Abwassers an organischen Abfällen so groß, daß eine anaerobe Methanvergärung möglich wird. Es wird eine energieerzeugende Abwasserreinigung organisch, gering belasteter Abwässer geschaffen, bei der ein energiehaltiges Gas (Brennwert 20 bis 25 MJ/m³) produziert wird, das in vielfältiger Weise energetisch nutzbar ist. Auf die übliche energiebehaftete Sauerstoffzufuhr, wie sie bei aeroben Verfahren notwendig ist, kann verzichtet werden.

Die Zugabemenge der in der zweiten Reinigungsstufe anfallenden Biomasse und gegebenenfalls weiterer Biomasse, richtet sich nach der Belastung des ankommenden Abwassers. Dadurch ist es möglich, die Belastung des die erste Reinigungsstufe verlassenden Abwassers konstant zu halten. Die zweite Reinigungsstufe ist gegen Überbelastung geschützt, und es kommt nicht zum ungeklärten Durchlauf des Abwassers durch die zweite Reinigungsstufe.

Ist die Belastung des ankommenden Abwassers hoch, so wird ganz oder teilweise auf die Zuführung von Biomasse aus in der zweiten Reingungsstufe geernteter pflanzlicher Biomasse und weiterer zusätzlicher Biomasse verzichtet. Bei dieser Variante wird mit konstanter Durchlaufgeschwindigkeit in der ersten Reinigungsstufe gearbeitet.

Vorzugsweise beim erfindungsgemäßen Verfahren so gearbeitet, daß das die erste Reinigungsstufe verlassende Abwasser als Belastung einen CSB-Wert um 300 mg/l aufweist.

Ebenso ist es möglich, eine größere Belastung des Abwassers durch einen verlängerten Aufenthalt in der ersten Reinigungsstufe aufzufangen, um auf den gewünschten Belastungswert des die erste Reinigungsstufe verlassenden Abwassers zu kommen.

Mit dem erfindungsgemäßen Verfahren wird es möglich, die bei bisherigen Pflanzenkläranlagensystemen anfallende, nicht genutzte pflanzliche Biomasse im Reinigungsprozeß zu verwerten.

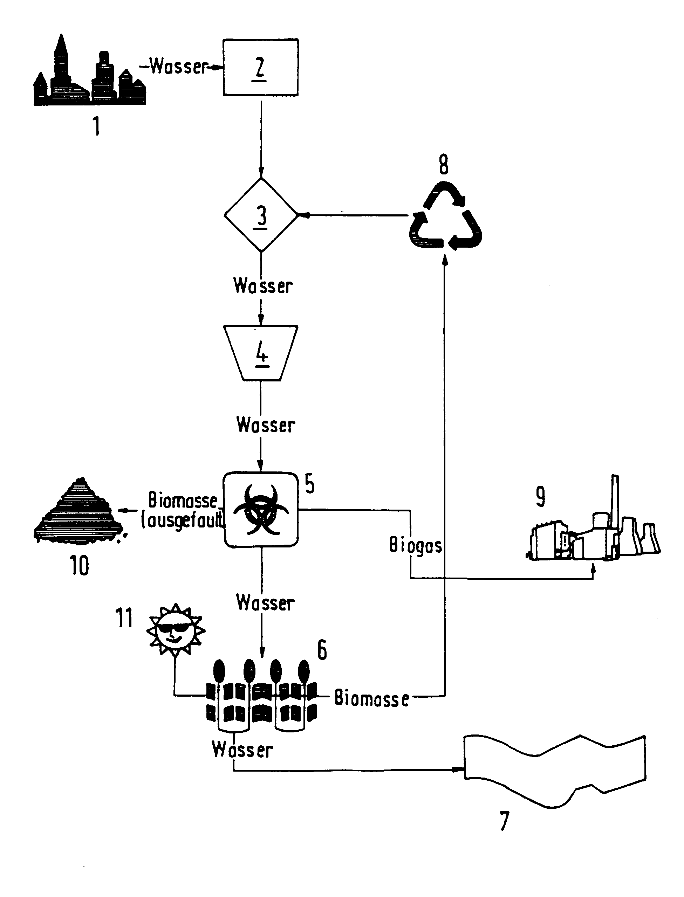

Die Erfindung wird anhand der Figur an zwei Prozeßvarianten erläutert. Selbstverständlich sind auch andere Ausführungsformen von der Erfindung erfaßt.

Die Prozeßvariante A bezieht sich auf eine Ausführungsform, bei der die zweite Reinigungsstufe als aquatische Pflanzenkläranlage ausgeführt wird.

Die Prozeßvariante B bezieht sich auf eine Ausführungsform, bei der die zweite Reinigunsstufe als eine Landbehandlung ausgeführt ist.

Allgemein wird das Abwasser von der Abwasserquelle 1 zur Abwasserreinigungsanlage transportiert. Dort werden in einem ersten Reinigungsschritt die gröberen und feineren Stoffe z. B. durch Sinkscheidung 2 mechanisch entfernt. Nach dieser Behandlung wird an der Dosierstelle 3 die zerkleinerte Biomasse aus dem Lager- und Zerkleinerungsbehälter 8 zugegeben und so die Belastung auf einen für die erste Reinigungsstufe optimalen Wert angehoben. Sinkstoffe werden nochmals im Sandfang 4 entfernt.

Der Lager- und Zerkleinerungsbehälter 8 wird zum einen durch die in der zweiten Reinigungsstufe gewonnene pflanzliche Biomasse und zum anderen durch zusätzliche organische Stoffe und Abfälle gespeist. Das belastungsoptimierte und mechanisch gereinigte Abwasser tritt nun in die erste Reinigungsstufe, den anaeroben Reaktor 5, ein. Dort wird die fäulnisfähige Biomasse abgeschieden und akkumuliert sich im Reaktor 5. Aus dem Reaktor 5 wird die ausgefaulte Biomasse kontinuierlich oder periodisch je nach Verfahrensweise des Reaktors 5 abgezogen und nach Restausfaulung einem weiteren Verwendungszweck 10 zugeführt. Das entstehende Biogas (Methananteil um 85%) wird abgezogen und energetisch bei einem Verbraucher 9 genutzt.

Das Abwasser tritt dann mit einem CSB-Gehalt um 300 mg/l aus der ersten Reinigungsstufe aus und dient in der zweiten Reinigungsstufe, einer Pflanzenkläranlage 6 zur Bewässerung und als natürlicher, organischer Dünger zur von Solarenergie 11 gespeister Produktion von Biomasse durch Fotosynthese.

In der Prozeßvariante A besteht die Pflanzenkläranlage 6 aus einer Teichanlage, die vorzugsweise grabenförmig, zur Vermeidung von Kurzschlußströmungen ausgestaltet ist. Für den pflanzlichen Besatz eignet sich die Wasserhyazynthe (Eichornia crassipes). Sie ist eine toxisch hochresistente Pflanze mit einer ausgeprägten Vermehrungsrate, ein 30%iger Besatz dehnt sich bei geeigneten, klimatischen Verhältnissen innerhalb eines Monats auf die Vollfläche aus und hohen Aufnahmeraten von Stickstoff und Schadstoffen.

In der Prozeßvariante B besteht die Pflanzenkläranlage 6 aus einer Landbehandlung, wobei es erfindungsgemäß keine Rolle spielt in welcher Richtung, horizontal oder vertikal, der Boden von dem Abwasser in der Reinigungsstufe durchströmt wird.

Vorteilhaft wird eine Oberflächenbehandlung des Bodens mit einer horizontalen und vertikalen Durchströmung bei dieser Variante gewählt. Es ist jedoch auch möglich, mit einer horizontalen oder vertikalen Durchströmung des Bodens zu arbeiten.

Für die Prozeßvariante bietet sich das St. Augustine Gras (Stenotaphrum secundatum) an. Es ist durch eine sehr starke vegetative Vermehrung, Salzresistenz und guter Verträglichkeit gegenüber Staunässe, auch über längere Zeit, geprägt. Bei der Landbehandlung wirkt sich außerdem die Filterwirkung des Bodens vorteilhaft aus.

Die vorher genannten Pflanzenarten stellen jedoch keine Ausschließlichkeit dar. Es ist möglich, andere Pflanzenarten als Besatz bei der zweiten Reinigungsstufe zu verwenden.

Nach Durchlaufen der zweiten Reinigungsstufe ist das Abwasser soweit gereinigt, daß es den Anforderungen an normale Einleitungswerte erfüllt und direkt in den Vorfluter 7 eingeleitet werden oder einer anderen Verwendung zukommen kann.

Mit dem erfindungsgemäßen Verfahren ist es möglich, bei entsprechender Dimensionierung der Reinigungsstufen oder aber Einstellung der Durchlaufzeit des Abwassers durch die erste Reinigungsstufe im Ablaufwasser der zweiten Reinigungsstufe Ablaufwerte zu erhalten, die den konventionellen Kläranlagen entsprechen oder diese gar unterbieten.

## Patentansprüche

1. Verfahren zur Reinigung von Abwasser, wobei eine zweistufige Verfahrensführung eingesetzt wird, die zum einen aus einer anaeroben Behandlung in der ersten Reinigungsstufe mit Produktion von methanhaltigem Biogas und einer Behandlung in einer Pflanzenkläranlage als zweiter Reinigungsstufe besteht und die zum anderen die in der zweiten Reinigungsstufe geerntete Biomasse vor der ersten Reinigungsstufe in das Abwasser einbringt.

2. Verfahren nach Anspruch 1, **dadurch gekennzeichnet**, daß zusätzlich andere organische Stoffe und Abfälle vor der ersten Reinigungsstufe in das Abwasser eingebracht werden.

3. Verfahren nach Anspruch 1 und 2, **dadurch gekennzeichnet**, daß die Belastung an organischer Biomasse des aus der ersten Stufe austretenden Abwassers konstant gehalten wird.

4. Verfahren nach Anspruch 1 bis 3, **dadurch gekennzeichnet**, daß die Belastung an organischer Biomasse des aus der ersten Stufe austretenden Abwassers durch die Zugabemenge an geernteter Biomasse aus der zweiten Reinigungsstufe und/oder zusätzlichen organischen Stoffen und Abfällen in das Abwasser vor der ersten Reinigungsstufe gesteuert wird.

5. Verfahren nach Anspruch 1 bis 3, **dadurch gekennzeichnet**, daß die Belastung des aus der ersten Reinigungsstufe austretenden Abwassers durch die Verweilzeit des Abwassers in der ersten Reinigungsstufe gesteuert wird.

## Claims

1. Process for cleaning of waste water in which a two stage cleaning method is introduced which consists of an anaerobic treatment in a first cleaning stage with the production of methane containing biogas and a treatment in a plant clarifying unit as a second cleaning stage and which furthermore introduces out the second cleaning stage biomass settled into from the first cleaning stage in the waste water.

2. Process according to claim 1, **characterised in that** additional other organic material and waste is introduced into the waste water before the first cleaning stage.

3. Process according to claim 1 and 2, **characterised in that** the loading of organic biomass of the waste water exiting from the first stage is maintained constant.

4. Process according to claim 1 to 3, **characterised in that** the loading of organic biomass in the waste water from the first stage is controlled by the added quantity of biomass gathered from the second cleaning stage and/or additional organic materials and waste in the waste water before the first cleaning stage.

5. Process according to claim 1 to 3, **characterised in that** the loading of the waste water exiting from the first cleaning stage is controlled by the dwell time of the waste water in the first cleaning stage.

## Revendications

1. Procédé pour l'épuration des eaux usées, dans lequel on met en oeuvre un mode opératoire en deux étapes qui consiste d'une part en un traitement anaérobie dans la première étape de nettoyage avec production de biogaz contenant du méthane et un traitement dans une station d'épuration végétale comme deuxième étape de nettoyage et qui d'autre part introduit dans les eaux usées la biomasse recueillie dans la deuxième étape de nettoyage avant la première étape de nettoyage.

2. Procédé selon la revendication 1, caractérisé en ce que de plus on introduit d'autres matières organiques et déchets avant la première étape de nettoyage dans les eaux usées.

3. Procédé selon la revendication 1 et 2, caractérisé en ce que la charge en biomasse organique des eaux usées provenant de la première étape est maintenue constante.

4. Procédé selon la revendication 1 à 3, caractérisé en ce que la charge en biomasse organique des eaux usées provenant de la première étape est régulée par la quantité d'addition en biomasse recueillie à partir de la deuxième étape de nettoyage et/ou en matières organiques supplémentaires et déchets dans les eaux usées avant la première étape de nettoyage.

5. Procédé selon la revendication 1 à 3, caractérisé en ce que la charge des eaux usées provenant de la première étape de nettoyage est régulée par le temps de séjour des eaux usées dans la première étape de nettoyage.
